# EUROPEAN PATENT APPLICATION

(11) **EP 4 066 838 A1**
(43) Date of publication of application: **05.10.2022**
(21) Application number: 20892653.5
(22) Date of filing: 26.11.2020
(51) Int. Cl.: A61K 31/506, A61P 29/00, C07D 239/48

(54) **COMPOSITION CONTAINING AROMATIC HETEROCYCLIC COMPOUND IN AMORPHOUS FORM, AND PREPARATION METHOD THEREFOR AND USE THEREOF**

(30) Priority: 26.11.2019 CN 201911171553
(71) Applicant: Shenzhen Chipscreen Biosciences Co., Ltd., Shenzhen, Guangdong 518057 (CN)
(72) Inventor: WANG, Shigang, Shenzhen, Guangdong 518057 (CN); SHAN, Song, Shenzhen, Guangdong 518057 (CN); YU, Jingdi, Shenzhen, Guangdong 518057 (CN); LIU, Xia, Shenzhen, Guangdong 518057 (CN); ZHAO, Chuantong, Shenzhen, Guangdong 518057 (CN); ZHANG, Yu, Shenzhen, Guangdong 518057 (CN); DENG, Xingyu, Shenzhen, Guangdong 518057 (CN); HUANG, Ning, Shenzhen, Guangdong 518057 (CN); WANG, Xianghui, Shenzhen, Guangdong 518057 (CN); PAN, Desi, Shenzhen, Guangdong 518057 (CN)
(74) Representative: Colombo, Stefano Paolo
(86) International application number: PCT/CN2020/131736
(87) International publication number: WO 2021/104363

(57) **Abstract**

Disclosed are a composition containing an aromatic heterocyclic compound in an amorphous form, and a preparation method therefore and a use thereof. Specifically, disclosed is a composition containing a compound of Formula (1) and a carrier, wherein the compound of formula (1) is in an amorphous form. The composition shows valuable properties in terms of in vivo absorption and bioavailability, and has the advantages of rapid absorption and high bioavailability, etc.

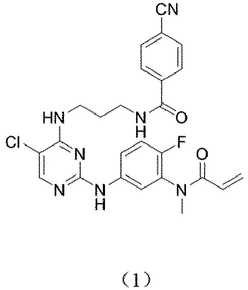

## Description

The present application is based on the application with the CN application number of 201911171553.2 and the filing date of November 26, 2019, and claims its priority. The disclosure of the CN application is hereby incorporated into this application in its entirety.

### Technical Field

The present invention relates to the field of pharmaceutical preparation, in particular to a composition containing an amorphous aromatic heterocyclic compound, a preparation method and use thereof.

### Background Art

Protein kinases are a class of intracellular messenger-dependent enzymes that catalyze the phosphorylation of specific proteins and complete the signal transmission process, mainly including tyrosine protein kinases (JAKs, Src, Abl, EGFR, FGFR, PDGFR, etc.), serine/threonine protein kinases (PKC, MAPK, Rho kinase, etc.), dual specificity protein kinases (MAPKK), and phosphatidylinositol kinases (PI3K). The phosphorylation/dephosphorylation processes catalyzed by protein kinases can regulate a variety of biological processes in different cells, such as metabolism, cell differentiation, cell survival, apoptosis, organ formation, angiogenesis, immune response, etc. (Shchemelinin I., et al. 2006, Folia Biol., 52: 81-100).

JAK kinases (Janus kinases, JAKs for short, including four known members JAK3, JAK1, TYK2, JAK2) are a small family in the cytoplasmic non-receptor tyrosine protein kinase superfamily. JAK3 is distributed in the bone marrow and lymphatic system, while JAK1, TYK2, and JAK2 are widely distributed in a variety of tissue cells. When JAKs bind to cytokine receptors on the cell surface, the receptor-coupled JAKs are activated, and then the receptors are phosphorylated, which provides a recruitment response site for the cytoplasmic signal transducers and activators of transcription (STAT proteins, including STAT1 to 4, STAT5a, STAT5b, STAT6), JAKs phosphorylate STAT proteins, which dimerize and transfer to the nucleus to regulate gene expression. This pathway is the JAK/STAT signaling pathway (O'Shea J. J. , et al. 2013, N. Engl. J. Med., 368: 161-170).

The JAK/STAT signaling pathway is a signal transduction pathway stimulated by a variety of cytokines and growth factor receptors, including interleukins (IL-2 to 7, IL-9, IL-10, IL-15, IL-21), interferons (IFN-α, IFN-β, IFN-γ), erythropoietin (EPO), granulocyte-macrophage colony-stimulating factor (GM-CSF), growth hormone (GH), prolactin (PRL), thrombopoietin (TPO), etc., which play a key role in the biological processes of immune cell and hematopoietic stem cell proliferation and immune regulation (Ghoreschi K., et al. 2009, Immunol. Rev., 228: 273-287). Different receptors can activate different subtypes of JAK kinases, thereby achieving differentiated biological functions.

CN 105399685 A discloses the following compound of Formula (1): 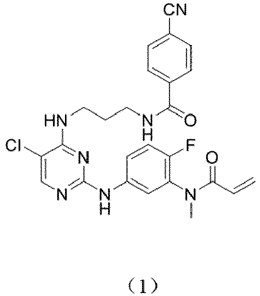 which has protein kinase inhibitory activity and can be used for the treatment and/or prevention of an autoimmune disease, inflammatory disease or cancer.

During the experiment, the inventor found that the compound of Formula (1) has problems such as low bioavailability. Hence, there is a realistic need to seek a suitable method to improve the clinical efficacy of the compound in the above-mentioned disease within a safe therapeutic window.

### Contents of the present invention

In order to solve the above problems, the inventors conducted in-depth research and found that when the compound of Formula (1) is loaded into the carrier of the present invention, and the compound of Formula (1) is in an amorphous form, the bioavailability of the compound can be significantly improved by further adding a suitable pharmaceutical excipient to prepare a tablet, capsule, granule, gel, and soft capsule. The present invention is hereby achieved on this basis.

Therefore, one object of the present application is to provide a composition comprising a compound of Formula (1) and a carrier, wherein the compound of Formula (1) is in an amorphous form,

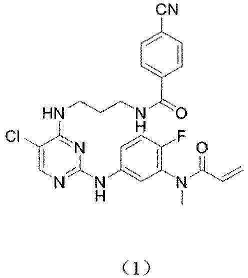

Another object of the present invention is to provide a method for preparing the composition.

A further object of the present invention is to provide a pharmaceutical composition, which comprises the composition; optionally, it further comprises one or more pharmaceutical excipients.

A further object of the present invention is to provide a method for preparing the pharmaceutical composition.

Another object of the present invention is to provide use of the composition or the pharmaceutical composition in the manufacture of a medicament for the treatment or prevention of an autoimmune disease, inflammatory disease or cancer.

The purpose of the present invention is realized through the following technical solutions:
The present invention provides a composition comprising the compound of Formula (1), which comprises the compound of Formula (1) and a carrier, wherein the compound of Formula (1) is in an amorphous form.

The term "amorphous form" or "amorphous" refers to a solid that exists in an amorphous state or form. Amorphous solids are of disordered arrangement of molecules and therefore have no distinguishable lattice or unit cell, no determinable long-range ordering. Solid form ordering of solids can be determined by standard techniques known in the art, for example by X-ray powder diffraction (XRPD) or differential scanning calorimetry (DSC). Amorphous solids can also be distinguished from crystalline solids, for example by birefringence using polarized light microscopy. The DSC curve of the composition of the present invention has no sharp characteristic peak. The characteristic of its X-ray powder diffraction pattern is that there is no crystal characteristic peak of the compound of Formula (1) after deducting the background peaks of the auxiliary material.

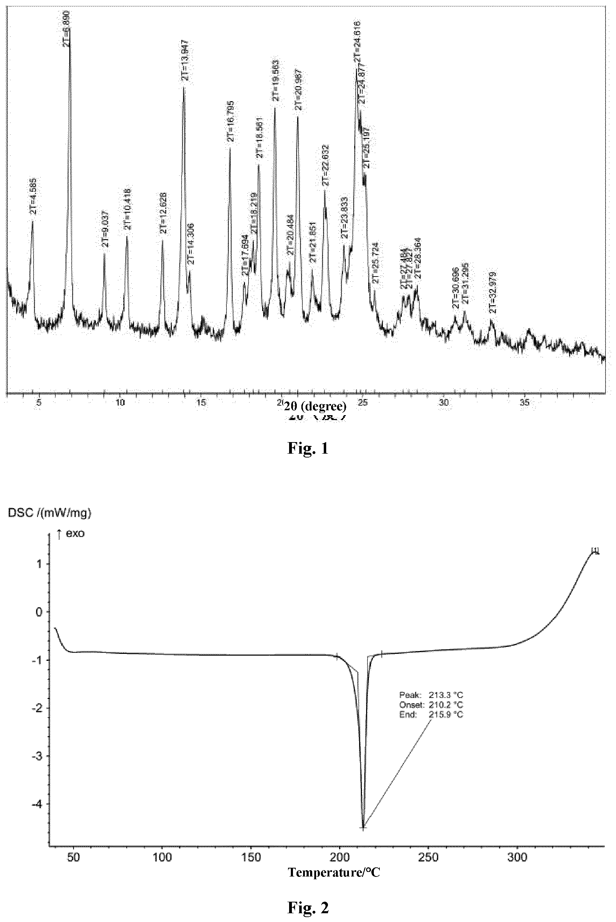

In some embodiments, the crystal of the compound of Formula (1) has characteristic peaks at least at the following positions expressed as 2θ in the X-ray powder diffraction pattern using Cu-Kα radiation: 6.9±0.2, 13.9±0.2, 19.6±0.2, 21.0±0.2 and 4.6±0.2. In some embodiments, it further has characteristic peaks at the following positions: 16.8±0.2, 18.6±0.2, 22.6±0.2, 24.9±0.2, and 25.2±0.2. In some embodiments, a typical X-ray powder diffraction pattern of the crystal of the compound of Formula (1) is shown in Figure 1.

In some embodiments, the carrier is selected from one or more of the following: a water-soluble carrier material, a poorly soluble carrier material and an enteric carrier material.

In some embodiments, the water-soluble carrier material is selected from the group consisting of a polyethylene glycol, a povidone, a surfactant, a polyvinyl alcohol, and a cellulose.

In some embodiments, the polyethylene glycol is selected from the group consisting of polyethylene glycol 2000, polyethylene glycol 4000, polyethylene glycol 6000, polyethylene glycol 8000, polyethylene glycol 10000, more preferably polyethylene glycol 6000 and polyethylene glycol 8000. In some embodiments, the povidone is selected from the group consisting of povidone K15, povidone K25, povidone K30, povidone K90, copovidone, and the like. In some preferred embodiments, the povidone is copovidone, preferably copovidone VA64. In some embodiments, the surfactant is selected from the group consisting of Soluplus, poloxamers, myrij (polyoxyethylene fatty acid ester), polyoxyethylene castor oil, sodium dodecylsulfate, phytantriol and polysorbate 80, etc., preferably poloxamers and sodium dodecylsulfate. In some embodiments, the polyvinyl alcohol is selected from the group consisting of polyvinyl alcohol and compositions of povidone-polyvinyl alcohol. In some embodiments, the cellulose is selected from the group consisting of hypromellose, hydroxypropylcellulose, hydroxyethylcellulose, and the like.

In some embodiments, the poorly soluble carrier material is selected from the group consisting of ethyl cellulose and a lipid-based material.

In some embodiments, the lipid-based material is selected from the group consisting of cholesterol, sitosterol, triethyl citrate, and glycerol monooleate.

The enteric carrier material refers to a carrier material that is hardly soluble or insoluble in water but soluble in an alkaline solution. In some embodiments, the enteric carrier material is selected from the group consisting of carboxymethylcellulose, hypromellose phthalate, hypromellose succinate, and a polyacrylic resin.

In some preferred embodiments, the carrier material is a water-soluble carrier material selected from one or more of the following:
a polyethylene glycol, preferably polyethylene glycol 2000, polyethylene glycol 4000, polyethylene glycol 6000, polyethylene glycol 8000, polyethylene glycol 10000, more preferably polyethylene glycol 6000 or polyethylene glycol 8000;
a povidone, preferably povidone K15, povidone K25, povidone K30, povidone K90 and copovidone, etc., more preferably copovidone, most preferably copovidone VA64;
a surfactant, preferably poloxamers, Myrij, polyoxyethylene castor oil, sodium dodecylsulfate, polysorbate 80, etc., more preferably poloxamers and sodium dodecylsulfate;
a polyvinyl alcohol, preferably polyvinyl alcohol and compositions of povidone-polyvinyl alcohol;
a cellulose, preferably hypromellose, hydroxypropylcellulose, hydroxyethylcellulose, and the like.

In some preferred embodiments, the carrier is selected from the group consisting of a povidone, hypromellose, hydroxypropylcellulose, Soluplus, phytantriol, glycerol monooleate and poloxamers, and the povidone comprises povidone K15, povidone K25, povidone K30 and povidone K90 and copovidone.

In some embodiments, the weight ratio of the compound of Formula (1) to the carrier is 1:2 to 1:20; for example, 1:2 to 1:10, such as, 1:2, 1:3, 1:4, 1 :5, 1:6, 1:7, 1:8, 1:9 or 1:10.

In some embodiments, in addition to the carrier, the composition may further comprise a solvent, a diluent, or a plasticizer. In some embodiments, the solvent is one or more in combination selected from the group consisting of water, methanol, ethanol, acetone, tetrahydrofuran, dichloromethane and dimethyl sulfoxide, preferably one or more in combination selected from the group consisting of water, ethanol, dichloromethane and tetrahydrofuran, more preferably water and/or ethanol.

In some embodiments, the composition may be further loaded to a matrix material to form a pellet (micropellet). In some embodiments, the pellet comprises a pellet core, a drug layer and a coating layer; wherein the drug layer comprises a compound of Formula (1) and a povidone-based carrier material, and the compound of Formula (1) is of amorphous type. In some embodiments, the povidone-based carrier material is selected from the group consisting of povidone K15, povidone K25, povidone K30 and povidone K90 and copovidone, preferably copovidone. In some embodiments, the weight ratio of the compound of Formula (1) to the povidone-based carrier material is 1:2 to 1:10; for example, 1:2, 1:3, 1:4, 1:5, 1 :6, 1:7, 1:8, 1:9 or 1:10; preferably 1:3. In some embodiments, the weight ratio of the pellet core, the drug layer and the coating layer is (5-8):(10-15):1, for example 20:40:3. In some embodiments, the pellet comprises a pellet core, a drug layer, and a coating layer; wherein the drug layer comprises the compound of Formula (1) in amorphous form and copovidone, wherein the weight ratio of the compound of Formula (1) to copovidone is 1:3, and the weight ratio of the pellet core, the drug layer and the coating layer is 20:40:3. The selection of the material of the pellet core and the coating layer, as well as the method for preparing the pellet are known in the art.

In some embodiments, the composition is prepared by a melting method or a solvent method.

The melting method refers to a method wherein the drug is mixed with the carrier uniformly, heated to melting; or the carrier material is first heated to melting, and then the drug is added thereto and mixed and dissolved. Under vigorous stirring, the melt is rapidly cooled into a solid or directly poured into a capsule for cooling, vacuum dried at room temperature or an appropriate temperature is conducted depending on the properties of the drug and the prepared composition, and taken out and pulverized after a certain period of time.

The solvent method refers to a method wherein the drug and the carrier are dissolved together in an organic solvent, or dissolved separately in the organic solvent and then mixed evenly, the organic solvent is removed by evaporation, the drug and the carrier are precipitated at the same time, and dried. Commonly used organic solvents are ethanol, acetone, etc.

Another aspect of the present application provides a method for preparing the composition, which comprises a step of preparing the composition by a melting method or a solvent method.

In some embodiments, the solvent method comprises the steps of:
the compound of Formula (1) together with the carrier and the solvent are placed in a water bath at 40-100°C (e.g., 60-90°C) and heated, dissolved under stirring or ultrasonic, and the solvent is removed (e.g., by rotary evaporation, spray drying, freeze drying, fluidized bed drying, or supercritical fluid method, etc.) to prepare the composition.

In some embodiments, the solvent is preferably used in the lowest amount that can completely dissolve the compound of Formula (1) at a suitable temperature.

In some embodiments, the melting method comprises the steps of:
the compound of Formula (1) is mixed with the carrier, melted (e.g., melted at 100-250°C), mixed uniformly, and then cooled to solidify and pulverized to obtain the composition.

If the drug is insoluble in the molten carrier, the drug can be first dissolved in a small amount of an appropriate organic solvent, then the molten carrier is added, stirred evenly, the organic solvent is evaporated, and the composition is prepared by rapid solidification at a low temperature according to the melting method.

In another aspect, the present application further provides a pharmaceutical composition, which comprises the composition; optionally, further comprises one or more pharmaceutical excipients.

In some embodiments, the pharmaceutical composition is a tablet, a capsule, a granule or a gel.

In some embodiments, the capsule is a hard capsule, soft capsule, sustained-release capsule, controlled-release capsule or enteric-coated capsule, preferably hard capsule.

In some embodiments, the filing material of the hard capsule is powder, granules, microtablets or micropellets, preferably microtablets or micropellets, more preferably micropellets.

In some embodiments, the micropellet comprises a pellet core, a drug layer and a coating layer; wherein the drug layer comprises the compound of Formula (1) and a povidone-based carrier material, and the compound of Formula (1) is in amorphous form. In some embodiments, the povidone-based carrier material is selected from the group consisting of povidone K15, povidone K25, povidone K30 and povidone K90 and copovidone, preferably copovidone. In some embodiments, the weight ratio of the compound of Formula (1) to the povidone-based carrier material is 1:2 to 1:10; for example, 1:2, 1:3, 1:4, 1:5, 1:6, 1:7, 1:8, 1:9 or 1:10; preferably 1:3. In some embodiments, the weight ratio of the pellet core, the drug layer and the coating layer is (5-8):(10-15): 1, for example 20:40:3. In some embodiments, the micropellet comprises a pellet core, a drug layer, and a coating layer; wherein the drug layer comprises the compound of Formula (I) in amorphous form and copovidone, and the weight ratio of the compound of Formula (1) to copovidone is 1:3, and the weight ratio of the pellet core, the drug layer and the coating layer is 20:40:3. The selection of the material of the pellet core and the coating layer, as well as the method for preparing the micropellet are known in the art.

The pharmaceutical excipients refer to the excipients and additives used in the production of drugs and the formulation of prescriptions; they are substances other than active ingredients that have been reasonably evaluated in terms of safety and are contained in pharmaceutical preparations. Pharmaceutical excipients may not only have the function of excipient, act as carrier and improve stability, but also have the important functions of solubilizing, hydrotrope-solubilizing, sustained and controlled release, and are important ingredients that may affect the quality, safety and effectiveness of drugs; and examples thereof include solvent, propellant, solubilizing agent, hydrotropic agent, emulsifier, colorant, binder, disintegrant, filler, lubricant, wetting agent, osmotic pressure regulator, stabilizer, glidant, flavoring agent, preservative, suspending agent, coating material, spicesflavers, anti-adhesive, chelating agent, penetration enhancer, pH adjuster, buffer, plasticizer, surfactant, foaming agent, defoaming agent, thickener, inclusion agent, moisturizer, absorbent, diluent, flocculant and deflocculant, filter aid, release blocker, etc. Appropriate pharmaceutical excipients can be added according to the type of pharmaceutical preparation to achieve the purpose of the present invention.

The commonly used filler (or diluent) includes, for example, lactose, glucose, pregelatinized starch, microcrystalline cellulose, starch, dextrin or mannitol, etc.; the commonly used binder (wetting agent) include, for example, water, ethanol, celluloses, povidone, starch syrup, dextrin, sugar and syrup or mucilage, etc.; the commonly used disintegrant includes, for example, crospovidone, low-substituted hydroxypropylcellulose, sodium hydroxymethylstarch, etc.; the commonly used lubricant (anti-sticking agent, glidant) includes, for example, stearic acid, calcium stearate, magnesium stearate, talc, hydrogenated vegetable oil, polyethylene glycol, sodium (magnesium) dodecylsulfonate, etc.

The unit dose of the pharmaceutical preparation is 5 to 200 mg.

The present invention also provides use of the composition or the pharmaceutical composition in the manufacture of a medicament for the treatment of a disease, wherein the disease is selected from the group consisting of autoimmune disease, inflammatory disease and cancer.

The present application also provides a method of treating a disease, comprising the step of administering to a subject in need thereof a therapeutically effective amount of the composition or pharmaceutical composition, wherein the disease is selected from the group consisting of autoimmune disease, inflammatory disease and cancer.

The application also provides the composition or pharmaceutical composition, for use in the treatment of a disease, wherein the disease is selected from the group consisting of autoimmune disease, inflammatory disease and cancer.

In some embodiments, the disease is selected from the group consisting of rheumatoid arthritis, psoriasis, Crohn's disease, systemic lupus erythematosus, multiple sclerosis, type I diabetes, allergic disease, chronic obstructive pulmonary disease, asthma, leukemia and lymphoma.

### Beneficial effects of the present invention

Compared with the compound of Formula (1) in the existing known crystal form, the composition or pharmaceutical composition containing the compound of the Formula (1) in amorphous form provided by the present invention has the following positive effects: the compound of Formula (1) of the composition and pharmaceutical composition exists in amorphous form, it shows valuable properties in terms of in vivo absorption and bioavailability, and has the advantages of rapid absorption and high bioavailability.

### Brief Description of the Drawings

Figure 1 shows the X-ray powder diffraction pattern of the compound of Formula (1) prepared in Example 1.
Figure 2 shows the DSC endothermic transition spectrum of the compound of Formula (1) prepared in Example 1.
Figure 3 shows the DSC endothermic transition pattern of the composition containing the compound of Formula (1) prepared in Example 3.
Figure 4 shows the X-ray powder diffraction pattern of the composition containing the compound of Formula (1) prepared in Example 7.
Figure 5 shows the results of the rat PK administration test of the compositions containing the compound of Formula (1) prepared in Example 3, Example 4 and Example 7.
Figure 6 shows the results of the pharmacokinetic test of the pharmaceutical preparation of the compound of Formula (1) in rats.
Figure 7 shows the results of the pharmacokinetic test of the pharmaceutical preparation of the compound of Formula (1) in beagle dogs.

### Specific Models for Carrying Out the present invention

The present invention is further described in detail below in conjunction with specific examples and with reference to data. It should be understood that these examples are intended to illustrate the present invention only and not to limit the scope of the present invention in any way. It should be particularly pointed out that all similar substitutions and modifications are obvious to those skilled in the art, and they are deemed to be included in the present invention. In the following examples, various procedures and methods not described in detail are conventional methods well known in the art.

### Example 1: Preparation of the compound of Formula (1)

According to the method disclosed in Example 7 of CN 105399685 A, the compound of Formula (1) was prepared, and its crystal form was detected. The results were shown in Figures 1 and 2.

### X-ray powder diffraction method:

Test unit: Analysis and Testing Center, Research Institute of Tsinghua University, Shenzhen
Test equipment: X-ray powder diffractometer (X Pert3 Powder, PANalytical, Netherlands)
Test basis: "Chinese Pharmacopoeia", 2015 edition, Part Four, General rule 0451 - Second method of X-ray diffraction
Test method: An appropriate amount of the product was taken and ground into fine powder, and the copper target experiment was performed by the X-ray diffraction method (General rule 0451, powder X-ray diffraction method), in which the collection range of diffraction data (2θ) was 3° to 60°.

### DSC differential scanning calorimetry:

Instrument: NSTSCH NETZSCH, initial temperature: 40°C, end temperature: 350°C, heating rate: 10°C/min.

### Example 2: Preparation of composition containing the compound of Formula (1) by solvent method

1 g of the compound of Formula (1) prepared in Example 1 and 16 g of polyethylene glycol 6000 were taken, added to 100 g of absolute ethanol and 100 g of acetone, and heated in a 60°C water bath to completely dissolve the compound of Formula (1) and polyethylene glycol 6000, spray drying was performed by using a spray-drying device at a spray-drying temperature set to 100°C to obtain an off-white solid powder. The X-ray powder diffraction analysis showed diffuse bun-shape peaks, and the compound of Formula (1) in the composition was amorphous.

### Example 3: Preparation of composition containing the compound of Formula (1) by melting method

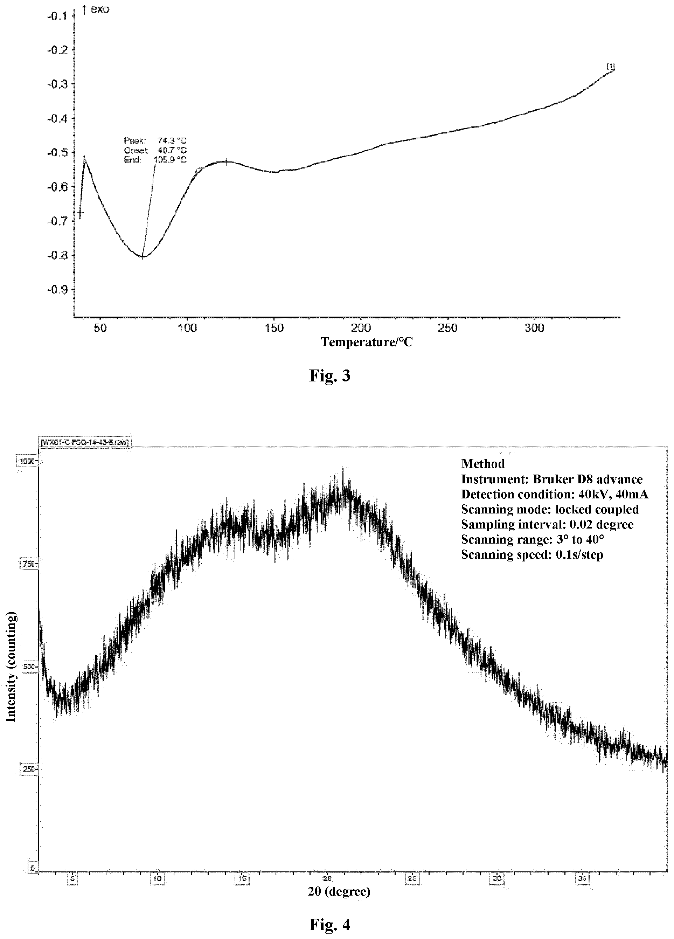

1 g of the compound of Formula (1) prepared in Example 1 and 5 g of povidone K30 were taken and mixed uniformly; and the compound of Formula (1) and povidone K30 were subjected to hot melt extrusion by using an extrusion temperature of 140°C, after the extrusion, the extruded strands were rapidly cooled and pulverized to obtain a composition containing the compound of Formula (1). The X-ray powder diffraction analysis showed that the compound of Formula (1) in the composition was amorphous. Its DSC endothermic transition spectrum was shown in Fig. 3.

### Example 4: Preparation of composition containing the compound of Formula (1) by solvent method

1g of the compound of Formula (1) prepared in Example 1 and 6g of hypromellose were taken, added to 100g of absolute ethanol and 100g of water, and heated in a water bath at 80°C to completely dissolve the compound of Formula (1) and hypromellose, spray-drying was performed by using a spray-drying device at a spray-drying temperature set to 120° C to obtain an off-white solid powder. The X-ray powder diffraction analysis showed that the compound of Formula (1) in the composition was amorphous.

### Example 5: Preparation of composition containing the compound of Formula (1) by solvent method

1 g of the compound of Formula (1) prepared in Example 1 and 2 g of sodium dodecylsulfate were taken, added to 160 g of absolute ethanol and 40 g of water, and heated in a water bath at 90°C to completely dissolve the compound of Formula (1) and sodium dodecylsulfate, spray-drying was performed by using a spray-drying device at a spray-drying temperature set to 120°C to obtain an off-white solid powder. The X-ray powder diffraction analysis showed that the compound of Formula (1) in the composition was amorphous.

### Example 6: Preparation of composition containing the compound of Formula (1) by melting method

8g of polyvinyl alcohol was taken, mixed evenly, melted at 240°C, added with 1g of the compound of Formula (1) prepared in Example 1 and 4g of mannitol, stirred to melt, rapidly cooled to form a solid, which was pulverized to obtain a composition containing the compound of Formula (1). The X-ray powder diffraction analysis showed that the compound of Formula (1) in the composition was amorphous.

### Example 7: Preparation of composition containing the compound of Formula (1) by solvent method

1 g of the compound of Formula (1) prepared in Example 1 and 3 g of copovidone VA64 were taken, added to 160 g of absolute ethanol and 40 g of water, and heated in a water bath at 70°C to completely dissolve the compound of Formula (1) and copovidone VA64, and rotary evaporation was performed at atmospheric pressure by a rotary evaporation method at a rotary evaporation temperature of 90°C to obtain an off-white solid powder. The X-ray powder diffraction analysis showed that the compound of Formula (1) in the composition was amorphous, and the results were shown in Fig. 4.

### Example 8: Preparation of composition containing the compound of Formula (1) by solvent method

1 g of the compound of Formula (1) prepared in Example 1, 8 g of ethylcellulose and 4 g of povidone K25 were taken, added to 200 g of absolute ethanol, and heated in a water bath at 70°C to completely dissolve the compound of Formula (1), ethylcellulose and povidone K25, and spray-drying was performed by using a spray-drying device at a spray-drying temperature set to 100°C to obtain an off-white solid powder. The X-ray powder diffraction analysis showed that the compound of Formula (1) in the composition was amorphous.

### Example 9: Preparation of composition containing the compound of Formula (1) by solvent method

1 g of the compound of Formula (1) prepared in Example 1, 12 g of acrylic resin L100, 1.2 g of triethyl citrate and 6.8 g of copovidone VA64 were taken, added to 200 g of absolute ethanol, and heated in a water bath at 70°C to completely dissolve the compound of Formula (1), acrylic resin L100 and copovidone VA64, and spray-drying was performed by using a spray-drying device at a spray-drying temperature set to 100°C to obtain an off-white solid powder. The X-ray powder diffraction analysis showed that the compound of Formula (1) in the composition was amorphous.

### Test Example 1: Pharmacokinetic test of compositions containing the compound of Formula (1) in rats

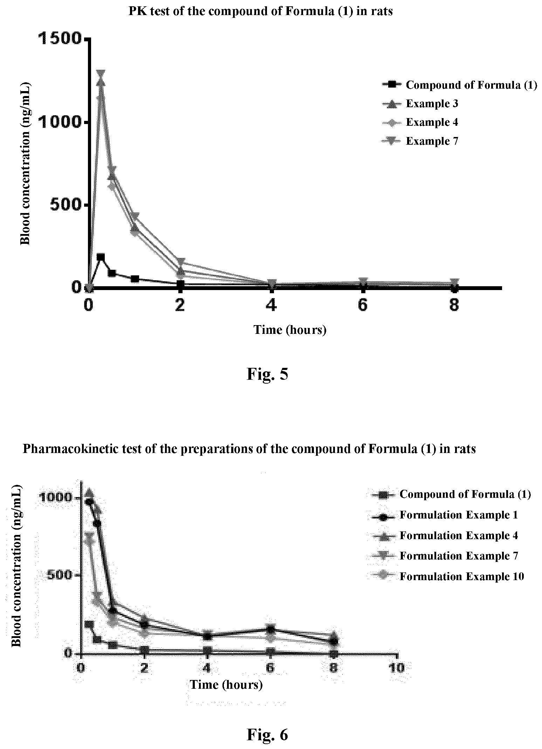

Twenty-four healthy rats were selected and randomly divided into 4 groups, 6 rats per group, which were respectively compound of Formula (1), Example 3, Example 4 and Example 7. During the experiment, the rats were fasted overnight before administration, and blood was collected from the orbit before administration, and the plasma was separated, which was taken as the blood concentration sample at 0h. The gavage solutions were prepared by dispersion with purified water, the concentration of the gavage solutions was 2 mg/ml, and each rat was administered with the compound of Formula (1) at a dose of 20 mg/kg, respectively. After administration, blood samples were collected at 15min, 0.5h, 1h, 2h, 4h, 6h, and 8h, and each sample was collected about 0.5ml, anticoagulated with heparin sodium, placed on ice after collection, and centrifuged within 1 hour to separate plasma, which was stored at -80°C for later test. The drug concentration in plasma was measured by LC-MS/MS. The test results were shown in Table 1 and Figure 5.

**Table 1: Comparison of pharmacokinetic data in rats**

| Parameter (ng/ml) | Compound of Formula (1) | Example 2 | Example 3 | Example 6 |
|---|---|---|---|---|
| AUC₍₀₋ₜ₎ | 235 | 1128 | 1134 | 1311 |
| Cmax | 243 | 1247 | 1130 | 1290 |

According to the test results in Table 1 and Figure 5, it could be seen that the compositions of Example 3, Example 4 and Example 7 could significantly improve the bioavailability of the compound of Formula (1) as compared with the active pharmaceutical ingredient of compound of Formula (1).

### Formulation Example 1: Capsules of the compound of Formula (1)

The drug-containing micropellets were prepared by loading the drug on the blank pellet cores using layering process in a fluid bed, and after being film coated, they were filled into vacant capsules to prepare capsules. The specific prescription and preparation process were as follows:
Prescription of capsules of the compound of Formula (1) (1000 capsules)

| process | material | amount (g) |
|---|---|---|
| loading the drug on pellet cores | blank pellet cores | 100.00 |
| | compound of Formula (1) | 50.00 |
| | copovidone | 150.00 |
| | 90% ethanol | 5000ml |
| film coating | premix for film coating | 15.00 |
| | purified water | 200ml |

### Preparation process:

(1) Preparation of drug solution: The compound of Formula (1) and copovidone were added to 90% ethanol solution, heated to dissolve at 80°C to form a transparent solution of the compound of Formula (1), wherein the compound of Formula (1) was amorphous;
(2) Loading the drug on pellet cores by layering process: The blank pellet cores were added to a fluid bed, and the compound of Formula (1) and copovidone were coated on the blank pellet cores by layering process in fluid bed to form drug-containing pellet cores;
(3) Preparation of film coating solution: The premix for film coating was added to the weighed purified water, and stirred for uniform dispersion to form a film coating solution;
(4) Film coating: the drug-containing pellet cores were added to a fluidized bed for film coating, and after completion, the micropellets were dried to remove water and ethanol to obtain micropellets of the compound of Formula (1);
(5) Capsule filling: The obtained micropellets of the compound of Formula (1) were filled into vacant capsules to prepare capsules of the compound of Formula (1).

### Formulation Example 2: Capsules of the compound of Formula (1)

A composition of the compound of Formula (1) and povidone was prepared by spray-drying process, granulated with other excipients by a dry method and then pelleted to form microtablets, and they were filled into vacant capsules to form capsules of the compound of Formula (1). The specific prescription and preparation process were as follows:
Prescription of capsules of the compound of Formula (1) (1000 capsules)

| process | material | amount (g) |
|---|---|---|
| spray-drying, dry granulation, pelleting | compound of Formula (1) | 25.00 |
| | povidone | 50.00 |
| | sodium carboxymethyl starch | 30.00 |
| | mannitol | 90.00 |
| | microcrystalline cellulose | 94.00 |
| | magnesium stearate | 1.00 |
| | 90% ethanol | 2500ml |
| film coating layer | premix for film coating | 8.80 |
| | purified water | 100ml |

### Preparation process:

(1) Preparation of a drug-containing solution: The compound of Formula (1) and povidone were added to 90% ethanol solution, heated to dissolve at 80°C to form a transparent solution of the compound of Formula (1);
(2) Spray-drying: The solution of the compound of Formula (1) and povidone was spray-dried to obtain a composition of the compound of Formula (1) and povidone;
(3) Dry granulation: The composition of the compound of Formula (1) and povidone was mixed with sodium carboxymethyl starch, mannitol, and microcrystalline cellulose evenly, and subjected to dry granulation to obtain granules;
(4) Blending: The granules obtained by dry granulation were blended with magnesium stearate;
(5) Pelleting to form microtablets: The mixed granules were subjected to pelleting to obtain microtablets;
(6) Film coating: The microtablets were loaded into a coating machine to perform film coating, and then the microtablets were dried to remove water to obtain microtablets of the compound of Formula (1);
(7) Capsule filling: The microtablets of the compound of Formula (1) were filled into vacant capsules to prepare capsules of the compound of Formula (1).

### Formulation Example 3: Capsules of the compound of Formula (1)

The drug-containing micropellets were prepared by loading the drug on the blank pellet cores using layering procedure in a fluid bed , and filled into vacant capsules to prepare capsules. The specific prescription and preparation process were as follows:
Prescription of capsules of the compound of Formula (1) (1000 capsules)

| process | material | amount (g) |
|---|---|---|
| loading the drug on pellet cores | blank pellet cores | 100.00 |
| | compound of Formula (1) | 50.00 |
| | hypromellose | 250.00 |
| | 75% ethanol | 5000ml |

### Preparation process:

(1) Preparation of drug solution: The compound of Formula (1) and hypromellose were added to 75% ethanol solution, and heated to dissolve at 80°C to form a transparent solution of the compound of Formula (1);
(2) Loading the drug on pellet cores by layering process: The blank pellet cores were added to a fluid bed, and the compound of Formula (1) and hypromellose were coated on the blank pellet cores by layering process in fluid bed to form drug-containing pellet cores;
(3) Drying micropellets: The drug-containing pellet cores were added into a fluid bed, the micropellets were dried to remove water and ethanol, thereby obtaining micropellets of the compound of Formula (1);
(4) Capsule filling: The obtained pellets of the compound of Formula (1) were filled into vacant capsules to prepare capsules of the compound of Formula (1).

### Formulation Example 4: Granules of the compound of Formula (1)

The composition of the compound of Formula (1) and hydroxypropyl cellulose was prepared by spray-drying process, and subjected to dry granulation with other excipients to prepare granules, which were filled into vacant capsules or packaged. The specific prescription and preparation process were as follows:
Prescription of capsules of the compound of Formula (1) (1000 capsules)

| process | material | amount (g) |
|---|---|---|
| spray-drying, dry granulation, blending, filling | compound of Formula (1) | 75.00 |
| | hydroxypropyl cellulose | 300.00 |
| | sodium carboxymethyl cellulose | 30.00 |
| | mannitol | 60.00 |
| | microcrystalline cellulose | 53.00 |
| | magnesium stearate | 2.00 |
| | 75% ethanol | 7500ml |

### Preparation process:

(1) Preparation of drug-containing solution: The compound of Formula (1) and hydroxypropyl cellulose were added to 75% ethanol solution, and heated to dissolve at 80°C to form a transparent solution of the compound of Formula (1);
(2) Spray-drying: The solution of the compound of Formula (1) and hydroxypropyl cellulose was subjected to spray-drying to obtain a composition of the compound of Formula (1) and hydroxypropyl cellulose;
(3) Dry granulation: The composition of the compound of Formula (1) and hydroxypropyl cellulose was mixed with sodium carboxymethyl cellulose, mannitol and microcrystalline cellulose evenly, and subjected to dry granulation to obtain granules;
(4) Blending: The granules obtained by dry granulation were blended with magnesium stearate;
(5) Capsule filling/packaging: The granules obtained by blending were filled with vacant capsules or packaged.

### Formulation Example 5: Granules of the compound of Formula (1)

The composition of the compound of Formula (1) and Soluplus (polyvinyl caprolactam-polyvinyl acetate-polyethylene glycol grafted copolymer) was prepared by hot-melt pulverization process, mixed with other excipients, and filled into vacant capsules or packaged. The specific prescription and preparation process were as follows:
Prescription of capsules of the compound of Formula (1) (1000 capsules)

| process | material | amount (g) |
|---|---|---|
| granulation, mixing, blending, filling | compound of the Formula (1) | 100.00 |
| | soluplus | 300.00 |
| | micronized silica gel | 30.00 |
| | magnesium stearate | 2.00 |
| | 90% ethanol | 10000ml |

### Preparation process:

(1) Preparation of drug-containing solution: The compound of Formula (1) and Soluplus were added to 90% ethanol solution, heated to dissolve at 80°C to form a transparent solution of the compound of Formula (1);
(2) Hot-melt pulverization: The solution of the compound of Formula (1) and Soluplus was subjected to hot-melt pulverization to obtain granules of the composition of the compound of Formula (1) and Soluplus;
(3) Particle mixing: The composition of the compound of Formula (1) and Soluplus was mixed with micronized silica gel uniformly;
(4) Blending: The obtained granules were blended with magnesium stearate;
(5) Particle filling/packaging: The granules obtained by blending were filled into vacant capsules or packaged.

### Formulation Example 6: Granules of the compound of Formula (1)

The compound of Formula (1) and copovidone and other excipients were subjected to granulation process by fluid bed to form granules, which were filled into vacant capsules or packaged. The specific prescription and preparation process were as follows:
Prescription of capsules of the compound of Formula (1) (1000 capsules)

| process | material | amount (g) |
|---|---|---|
| fluid bed granulation, blending, packaging | compound of Formula (1) | 25.00 |
| | copovidone | 250.00 |
| | crospovidone | 200.00 |
| | lactose | 800.00 |
| | microcrystalline cellulose | 715.00 |
| | magnesium stearate | 10.00 |
| | 90% ethanol | 2500ml |

### Preparation process:

(1) Preparation of drug-containing solution: The compound of Formula (1) and copovidone were added to 90% ethanol solution, and heated to dissolve at 80°C to form a transparent solution of the compound of Formula (1);
(2) Fluid bed granulation: Crospovidone, lactose, and microcrystalline cellulose were added to fluid bed, and the solution of the compound of Formula (1) and copovidone was sprayed therein for granulation to prepare drug-containing granules;
(3) Blending: The granules obtained by fluid bed granulation were blended with magnesium stearate;
(4) Packaging granules: The granules obtained by blending were packaged.

### Formulation Example 7: Tablets of the compound of Formula (1)

The composition of the compound of Formula (1) and hypromellose was prepared by spray-drying process, subjected to dry granulation with other excipients, then subjected to pelleting and film-coating to obtain tablets. The specific prescription and preparation process were as follows:
Prescription of tablets of the compound of Formula (1) (1000 tablets)

| process | material | amount (g) |
|---|---|---|
| dry-granulation, pelleting | compound of Formula (1) | 25.00 |
| | hypromellose | 125.00 |
| | low-substituted hydroxypropylcellulose | 50.00 |
| | mannitol | 140.00 |
| | microcrystalline cellulose | 158.00 |
| | magnesium stearate | 2.00 |
| | 70% ethanol | 2500ml |
| film-coating layer | premix for film-coating | 15 |
| | purified water | 200ml |

### Preparation Process:

(1) Preparation of drug-containing solution: The compound of Formula (1) and hypromellose were added to 70% ethanol solution, heated to dissolve at 80°C to form a transparent solution of the compound of Formula (1);
(2) Spray drying: The solution of the compound of Formula (1) and hypromellose was subjected to spray-drying to obtain the composition of the compound of Formula (1) and hypromellose;
(3) Dry granulation: The composition of the compound of Formula (1) and hypromellose was mixed with low-substituted hydroxypropyl cellulose, mannitol, and microcrystalline cellulose evenly, and subjected to dry granulation to obtain granules;
(4) Blending: The granules obtained by dry granulation were blended with magnesium stearate;
(5) Pelleting: The granules obtained by blending were subjected to pelleting to obtain plain tablets of the compound of Formula (1);
(6) Film coating: The plain tablets were added to a coating machine to perform film coating, and then the tablets were dried to remove water to obtain tablets of the compound of Formula (1).

### Formulation Example 8: Tablets of the compound of Formula (1)

The compound of Formula (1) and copovidone and other excipients were subjected to a granulation process by fluid bed to form granules, which were processed to form tablets. The specific prescription and preparation process were as follows:
Prescription of tablets of the compound of Formula (1) (1000 tablets)

| process | material | amount (g) |
|---|---|---|
| fluid bed granulation, pelleting | compound of Formula (1) | 50.00 |
| | copovidone | 150.00 |
| | crospovidone | 60.00 |
| | lactose | 400.00 |
| | microcrystalline cellulose | 136.00 |
| | magnesium stearate | 4.00 |
| | 90% ethanol | 5000ml |

### Preparation process:

(1) Preparation of drug-containing solution: The compound of Formula (1) and copovidone were added to 90% ethanol solution, and heated to dissolve at 80°C to form a transparent solution of the compound of Formula (1);
(2) Fluid bed granulation: Crospovidone, lactose, and microcrystalline cellulose were added to a fluid bed, and the solution of the compound of Formula (1) and copovidone was sprayed therein for granulation to obtain drug-containing granules;
(3) Blending granules: The granules obtained by fluidized bed granulation were blended with magnesium stearate;
(4) Pelleting: The granules obtained by blending were subjected to pelleting.

### Formulation Example 9: Gel of the compound of Formula (I)

The gel of the compound of Formula (1) was prepared by ultrasonic and stirring process. The specific prescription and preparation process were as follows:
Prescription of gel of the compound of Formula (1) (130g)

| process | material | amount (g) |
|---|---|---|
| rotary evaporation, ultrasonication, stirring, filling | compound of Formula (1) | 1.00 |
| | phytantriol | 20.00 |
| | poloxamer | 2.00 |
| | carbomer | 2.00 |
| | absolute ethanol | 100ml |
| | water | 100 |

### Preparation process:

(1) Preparation of drug-containing phase: The compound of Formula (1) and phytantriol were added to absolute ethanol solution, and heated to dissolve at 80°C to form a transparent solution of the compound of Formula (1), and then subjected to rotary evaporation at 80°C at atmospheric pressure to reach 30g;
(2) Preparation of poloxamer phase: Poloxamer was added to water and heated to dissolve in a water bath at 80°C;
(3) Mixing two phases: The drug-containing phase was slowly added dropwise to the poloxamer phase when they were still hot, the dropwise addition was performed under stirring, rapid stirring was performed by a magnetic stirrer for 3 hours, and then ultrasonication was carried out on a ultrasonic wave meter for 5 minutes;
(4) Preparation of gel: Carbomer was added to the mixed solution, stirred to dissolve to form a gel;
(5) Filling gel into bottles/tubes: The prepared gel was filled into bottles or tubes.

### Formulation Example 10: Soft capsules of the compound of Formula (1)

The filling liquid for the soft capsules of the compound of Formula (1) was prepared by rotary evaporation process. The specific prescription and preparation process were as follows:
Prescription of capsules of the compound of Formula (1) (50 capsules)

| process | material | amount (g) |
|---|---|---|
| stirring, rotary evaporation, filling | compound of Formula (1) | 1.00 |
| | glycerol monooleate | 20.00 |
| | poloxamer | 2.00 |
| | absolute ethanol | 100ml |

### Preparation process:

(1) Preparation of drug-containing solution: The compound of Formula (1) was added to absolute ethanol solution, and heated to dissolve at 80°C to form a transparent solution of the compound of Formula (1);
(2) Preparation of mixed solution: Glycerol monooleate was added to the drug-containing solution and mixed well, then added with poloxamer, and heated to dissolve in a water bath at 80°C;
(3) Rotary evaporation of the mixed solution: The mixed solution was subjected to rotary evaporation to reach 50ml, thereby forming a filling solution for soft capsules;
(5) Filling soft capsules: The obtained filling liquid for soft capsules was subjected to filling to obtain soft capsules of the compound of Formula (1).

### Formulation Example 11: Capsules of the compound of Formula (1)

The drug-containing micropellets were prepared by loading the drug on the blank pellet cores using layering procedure in a fluid bed, subjected to film coating, and then filled into vacant capsules to prepare capsules. The specific prescription and preparation process were as follows:
Prescription of capsules of the compound of Formula (1) (1000 capsules)

| process | material | amount (g) |
|---|---|---|
| loading the drug on pellet cores | blank pellet cores | 100.00 |
| | compound of Formula (1) | 50.00 |
| | copovidone | 150.00 |
| | water | 5000ml |
| film-coating | premix for film coating | 15.00 |
| | purified water | 200ml |

### Preparation process:

(1) Preparation of drug-containing solution: Copovidone was taken and added to water, stirred to dissolve completely, the compound of Formula (1) was taken and added to the aqueous solution of copovidone, stirred to fully disperse, thereby forming a suspension of the compound of Formula (1), in which the compound of Formula (1) was of a crystalline form;
(2) Loading drug on pellet cores by layering process: Blank pellet cores were added to a fluid bed, and the compound of Formula (1) and copovidone were coated on the blank pellet cores by layering process with the fluid bed to form drug-containing pellet cores;
(3) Preparation of film coating solution: Premix for film coating was added to the weighed purified water, stirred to uniformly disperse, thereby forming a film coating solution;
(4) Film coating: the drug-containing pellet cores were added to a fluid bed to perform film coating, and then the micropellets were dried to remove water, thereby obtaining micropellets of the compound of Formula (1);
(5) Capsule filling: The obtained micropellets of the compound of Formula (1) were filled into vacant capsules to obtain capsules of the compound of Formula (1).

### Test Example 2: Pharmacokinetic test of pharmaceutical preparations in rats

Thirty healthy rats were selected and randomly divided into 5 groups, 6 rats (3 females, 3 males) per group, which were respectively the groups of the compound of Formula (1), the capsules of Formulation Example 1, the granules of Formulation Example 4, the tablets of Formulation Example 7, and the soft capsules of Formulation Example 10. During the experiment, the rats were fasted overnight before administration, and blood was collected from the orbit before administration, and the plasma was separated, which was taken as the blood concentration sample at 0h. The gavage solutions were prepared by dispersing with purified water, the concentration of the gavage solutions was 2 mg/ml, and each rat was administered with the compound of Formula (1) at a dose of 20 mg/kg respectively. After administration, blood samples were collected at 15min, 0.5h, 1h, 2h, 4h, 6h, and 8h, and each sample was collected about 0.5ml, anticoagulated with heparin sodium, placed on ice after collection, and centrifuged within 1 hour to separate plasma, which was stored at -80°C for later test. The drug concentration in plasma was measured by LC-MS/MS. The test results were shown in Table 2 and Fig. 6.

**Table 2: Comparison of pharmacokinetic data in rats**

| Parameter (ng/ml) | Compound of Formula (1) | Formulation Example 1 | Formulation Example 4 | Formulation Example 7 | Formulation Example 10 |
|---|---|---|---|---|---|
| AUC₍₀₋ₜ₎ | 235 | 1656 | 1859 | 1385 | 1140 |
| Cmax | 243 | 975 | 1071 | 755 | 722 |

According to the test results in Table 2 and Fig. 6, it could be seen that, compared with the compound of Formula (1), the capsules of Formulation Example 1, the granules of Formulation Example 4, the tablets of Formulation Example 7 and the soft capsules of Formulation Example 10 showed significantly improved absorption of the compound of Formula (1).

In order to further verify that the preparations of the compound of Formula (1) could indeed improve the bioavailability of the compound of Formula (1), we carried out the pharmacokinetic study of the capsules of Formulation Example 1 in beagle dogs.

### Test Example 3: Pharmacokinetic test of pharmaceutical preparations in beagle dogs

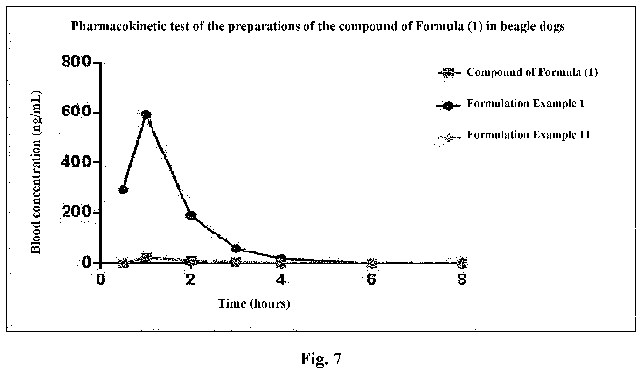

Nine healthy beagle dogs were selected, which were divided into the group of the compound of Formula (1), capsules of Formulation Example 1, and capsules of Formulation Example 11. During the experiment, the beagle dogs were fasted overnight before administration, and venous blood was collected before administration, and plasma was separated as the blood drug concentration sample at 0h. Each beagle dog was administrated by gavage at a dose of 100 mg of the compound of Formula (1), respectively. After administration, blood samples were collected at 15min, 0.5h, 1h, 2h, 4h, 8h, and 24h respectively. Each sample was collected about 0.5ml, anticoagulated with heparin sodium, placed on ice after collection, and centrifuged within 1 hour to separate plasma, the drug concentration in plasma was measured by LC-MS/MS. The test results were shown in Table 3 and Fig. 7.

**Table 3: Determination of drug concentrations (ng/ml) in beagle dog plasma at different time points by LC-MS/MS**

| Test animal | Cmax (ng/mL) | t_{1/2} (h) | AUC_{(0-T)} (h^{∗}ng/mL) |
|---|---|---|---|
| Compound of Formula (1) | 22 | 0.4 | 30 |
| Formulation Example 1 | 596 | 0.5 | 870 |
| Formulation Example 11 | 24 | 0.4 | 34 |

According to the test results in Table 3 and Figure 7, it could be seen that the capsules of Formulation Example 1 showed good absorption in beagle dogs.

Although specific embodiments of the present invention have been described in detail, it will be understood by those skilled in the art that, in light of all the teachings disclosed, various modifications and substitutions of those details may be made, and all of which fall within the scope of the present invention. The full scope of the present invention is given by the appended claims and any equivalents thereof.

## Claims

1. A composition, which comprises a compound of Formula (1) and a carrier, wherein the compound of Formula (1) is in an amorphous form,

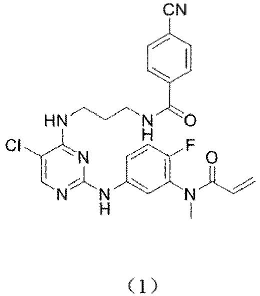

2. The composition according to claim 1, wherein the carrier is one or more selected from the following: a water-soluble carrier material, a poorly soluble carrier material and an enteric carrier material; preferably a water-soluble carrier material;
preferably, the water-soluble carrier material is selected from the group consisting of a polyethylene glycol, a povidone, a surfactant, a polyvinyl alcohol, a cellulose, carbomer and mannitol; preferably, the polyethylene glycol is selected from the group consisting of polyethylene glycol 2000, polyethylene glycol 4000, polyethylene glycol 6000, polyethylene glycol 8000 and polyethylene glycol 10000; preferably, the povidone is selected from the group consisting of povidone K15, povidone K25, povidone K30, povidone K90 and copovidone; preferably, the surfactant is selected from the group consisting of Soluplus, poloxamer, Myrij, polyoxyethylene castor oil, sodium dodecylsulfate, and polysorbate 80; preferably, the polyvinyl alcohol is selected from the group consisting of polyvinyl alcohol and a composition of povidone-polyvinyl alcohol; preferably, the cellulose is selected from the group consisting of hypromellose, hydroxypropyl cellulose and hydroxyethyl cellulose;
preferably, the poorly soluble carrier material is selected from the group consisting of ethyl cellulose and a lipid-based material; preferably, the lipid-based material is selected from the group consisting of cholesterol, sitosterol, triethyl citrate and glycerol monooleate;
preferably, the enteric carrier material is selected from the group consisting of carboxymethyl cellulose, hypromellose phthalate, hypromellose succinate and a polyacrylic resin; preferably, the polyacrylic resin is selected from Acrylic Resin L100.

3. The composition according to claim 1 or 2, wherein the carrier is selected from the group consisting of a povidone, hypromellose, hydroxypropyl cellulose, Soluplus, phytantriol, glycerol monooleate and poloxamer;
preferably, the povidone is selected from the group consisting of povidone K15, povidone K25, povidone K30, povidone K90 and copovidone.

4. The composition according to any one of claims 1 to 3, wherein the weight ratio of the compound of Formula (1) to the carrier is 1:2 to 1:20; for example, 1:2 to 1:10, and for a further example, 1 :2 to 1:6.

5. A method for preparing the composition according to any one of claims 1 to 4, comprising the steps of:
heating the compound of Formula (1) together with the carrier and a solvent in a water bath at 40°C to 100°C (e.g., 60°C to 90°C), dissolving (e.g., under stirring or ultrasonication), removing the solvent (e.g., by rotary evaporation, spray-drying, freeze-drying, fluidized bed drying or supercritical fluid method) to prepare the composition.

6. A method for preparing the composition according to any one of claims 1 to 4, comprising the steps of:
mixing the compound of Formula (1) and the carrier, melting (e.g., melting at 100°C to 250°C), mixing uniformly, and then cooling to solidify and pulverizing to obtain the composition.

7. A pharmaceutical composition, which comprises the composition according to any one of claims 1 to 4; optionally, further comprises one or more pharmaceutical excipients; preferably, the pharmaceutical composition is a tablet, capsule, granule or gel;
preferably, the capsule is hard capsule, soft capsule, sustained-release capsule, controlled-release capsule or enteric-coated capsule, preferably hard capsule;
more preferably, the filling material of the hard capsule is in form of powder, granules, microtablets or micropellets, preferably microtablets and micropellets, more preferably micropellets.

8. The pharmaceutical composition according to claim 7, the micropellet comprises a core, a drug layer and a coating layer; wherein the drug layer comprises the compound of formula (I) in amorphous form and copovidone, and the weight ratio of the compound of Formula (1) to copovidone is 1:2 to 1:10; and the weight ratio of the pellet core, the drug layer and the coating layer is (5-8) : (10-15) : 1.

9. The pharmaceutical composition according to claim 7 or 8, which has a unit dose of 5 to 200 mg.

10. Use of the composition according to any one of claims 1 to 4 or the pharmaceutical composition according to any one of claims 7 to 9 in the manufacture of a medicament for the treatment or prevention of a disease, wherein the disease is selected from the group consisting of autoimmune disease, inflammatory disease and cancer;
preferably, the disease is selected from the group consisting of rheumatoid arthritis, psoriasis, Crohn's disease, systemic lupus erythematosus, multiple sclerosis, type I diabetes, allergic disease, chronic obstructive pulmonary disease, asthma, leukemia and lymphatic tumor.
